# EUROPEAN PATENT SPECIFICATION

(11) **EP 3 004 318 B1**
(45) Date of publication and mention of the grant of the patent: **09.10.2019**
(21) Application number: 14732491.7
(22) Date of filing: 19.05.2014
(51) Int. Cl.: C12M 1/00, C12M 1/32, G01N 1/16

(54) **ASEPTIC SAMPLING MODULE AND MANIFOLD**
ASEPTISCHES PROBENENTNAHMEMODUL UND VERTEILER
MODULE D'ÉCHANTILLONNAGE ET DISTRIBUTEUR ASEPTIQUES

(30) Priority: 06.06.2013 US 201361832101 P; 30.01.2014 US 201461933806 P
(43) Date of publication of application: 13.04.2016
(73) Proprietor: Bend Research, Inc., Bend, OR 97701 (US)
(72) Inventor: NEWBOLD, David, Dixon, Bend, OR 97701 (US)
(74) Representative: De Clercq & Partners
(86) International application number: PCT/US2014/038614
(87) International publication number: WO 2014/197192

(56) References cited:
- WO-A1-2012/087235
- US-A1- 2005 217 351
- US-A1- 2012 137 793

## Description

### FIELD

The present disclosure is directed to an automatic aseptic sampling module and manifold and methods of using the same.

### BACKGROUND

Obtaining samples from containers or other systems that support biologically and/or chemically active environments can require complex and careful sampling procedures to avoid contamination of the containers or the environment itself. For example, most bioreactors require frequent sampling (e.g., one or more times a day) to monitor and control the conditions and levels of nutrients needed for cell growth. To reduce the risk of contamination within such systems, conventional sampling techniques generally require operators to perform multiple, labor-intensive steps.

In addition, samples from one aseptic source are typically directed to several analytical devices to measure various properties of the contents of the source, such as pH, dissolved oxygen, osmolality, nutrient concentrations, ammonia/ammonium, lactate/lactic acid, pCO₂, electrolytes (such as K+, Ca++, and/or Na+), amino acids, NAD/NADH, impurities, purity, phenotypes, metabolic states, cell cycle, or other properties US2012137793 discloses a sample collection device comprising an input conduit connected to a fluid processing line, a plurality of automated sample collection bags and a waste collection bag.

A valve block having a plurality of three-way valves and an output, said plurality of three-way valves corresponding in number to said plurality of sample collection bags, each of said three-way valves placing said input conduit in fluid communication with a different one of said plurality of sample collection bags, said output being in fluid communication with said waste collection bag.

WO2012087235 discloses a milk sampling system comprising a fluid conveying device for forwarding discrete samples of the milk to respective discharge paths for analysis. The fluid conveying device has an inlet connected to an induction system and an outlet connected to a discharge system, the induction system having supply paths arranged to selectively connect the pump inlet to a plurality of fluid sources including a milk source, and the discharge system having a plurality of discharge paths arranged to successively deliver discrete milk samples to the respective discharge paths for analysis.

US2005217351 discloses a sample-directing manifold for an automated sampling system comprising a plurality of sample inlets wherein each sample inlet is in fluid communication with a sample outlet valve and a waste outlet valve.

### SUMMARY

The aseptic sampling module and manifold disclosed herein provides consistent or substantially consistent sampling procedures for obtaining samples of a desired quality, while reducing the risk of contamination of the aseptic fluids and directing them to appropriate analytical devices. In some embodiments, samples from multiple sources may be directed to multiple analytical devices for analysis.

The sample-directing manifold comprises (a) a plurality of sample inlets, wherein each sample inlet is in fluid communication with a respective sample outlet valve and a respective waste outlet valve; (b) a sample outlet path in fluid communication with each respective sample outlet valve; and (c) a waste outlet path in fluid communication with each said respective waste outlet valve.

The sample-directing manifold is arranged such that each respective waste outlet valve and each respective sample outlet valve may be configured independently so that only one of the plurality of sample inlets may be in fluid communication with the sample outlet path.

In one embodiment, the sample-directing manifold further comprises a flushing fluid inlet in fluid communication with the sample outlet path.

In one embodiment, the sample-directing manifold is made from a body, the body having internal channels defining the plurality of sample inlets, the sample outlet path, and the waste outlet path. In another embodiment the body comprises channels for each of the respective sample outlet valves and each of the respective waste outlet valves.

Disclosed is a sampling system for collecting a fluid sample from an enclosed container comprises the sample-directing manifold. The sampling system further comprises an aseptic sampling valve. In one embodiment the aseptic sampling valve comprises a variable volume reservoir.

In one embodiment, the sampling system comprises a control module in communication with the sample-directing manifold and the aseptic sampling valve.

In another embodiment, a sample manifold can further comprise a gas inlet valve, a fluid inlet valve, and an outlet path isolation valve. Wherein the sample manifold can be configured so that each sample inlet can be connected to the sample outlet path through the sample outlet valve while the outlet path isolation valve is closed, while the other sample inlets are connected to the waste outlet path through the waste outlet valves, and wherein the fluid inlet can be configured to remove the sample from the sample outlet path when the outlet path isolation valve is open so as to direct the fluid to waste, and the gas inlet can be used to remove the fluid from the outlet path when the isolation valve is open, so as to prepare the outlet path for receiving another sample.

In another embodiment, a sampling system can be used for collecting a fluid sample from an enclosed container and comprises at least two modules, a control module, and a sampling module. The control module comprises (1) a source of compressed gas, (2) one or more valves for directing compressed gas to the sampling module, and (3) an optional vacuum pump. The sampling module comprises: (4) a sanitizing fluid inlet valve; (5) a gas inlet valve; (6) a sample collection valve; (7) an outlet valve; (8) a variable volume reservoir; and (9) a fluid flow path interconnecting (4) - (8).

In another embodiment, A method of collecting a fluid sample from an aseptic container, comprises: providing a sampling system according to any of the precedent claims and opening a sanitant inlet valve and directing sanitant through a sample outlet path, discharging said sanitant through an outlet path isolation valve; closing the sanitant inlet valve, opening a gas inlet valve to remove sanitant from the sample outlet path; opening sample inlet and a sample outlet valve to direct the sample to an analytical device.

### BRIEF DESCRIPTION OF THE DRAWINGS

FIG. 1 illustrates a schematic view of the sampling system.
FIG. 2 illustrates schematic views of another embodiment of the sampling system.
FIG. 3 illustrates a schematic view of the sample-directing manifold.
FIG. 4 illustrates a schematic view of an outlet valve for directing sample to an outlet path. The waste outlet valve is essentially of the same design.
FIG. 5 illustrates a schematic view of a variation of the sampling system.
FIG. 6 illustrates a schematic view of another variation of the sampling system.
FIG. 7 illustrates a schematic view of another variation of the sampling system.
FIG. 8 illustrates a schematic view of another variation of the sampling system.

### DETAILED DESCRIPTION

Various embodiments of sampling module are disclosed herein. The following description is exemplary in nature and is not intended to limit the scope, applicability, or configuration of the invention in any way. Various changes to the described embodiment may be made in the function and arrangement of the elements described herein without departing from the scope of the invention.

As used in this application and in the claims, the singular forms "a," "an," and "the" include the plural forms unless the context clearly dictates otherwise. Additionally, the term "includes" means "comprises." Further, the term "coupled" or "in communication with" generally means electrically, electromagnetically, and/or physically (e.g., mechanically or chemically) coupled or linked and does not exclude the presence of intermediate elements between the coupled or associated items absent specific contrary language. In some embodiments the elements include pneumatic coupling using a gas or other fluid.

The terms "upstream" and "downstream" are not absolute terms; instead, those terms refer to the direction of flow of fluids within a channel or pathway. Thus, with regard to a structure through which a fluid flows, a first area is "upstream" of a second area if the fluid flows from the first area to the second area. Likewise, the second area can be considered "downstream" of the first area.

Unless otherwise indicated, all numbers expressing quantities of ingredients, properties such as molecular weight, percentages, measurements, distances, ratios, and so forth, as used in the specification or claims are to be understood as being modified by the term "about." Accordingly, unless otherwise indicated, implicitly or explicitly, the numerical parameters set forth are approximations that may depend on the desired properties sought and/or limits of detection under standard test conditions/methods. When directly and explicitly distinguishing embodiments from discussed prior art, the embodiment numbers are not approximates unless the word "about" is recited.

Although the operations of exemplary embodiments of the disclosed method may be described in a particular, sequential order for convenient presentation, it should be understood that unless otherwise indicated, disclosed embodiments can encompass an order of operations other than the particular, sequential order disclosed. For example, operations described sequentially may in some cases be rearranged or performed concurrently. Further, descriptions and disclosures provided in association with one particular embodiment are not limited to that embodiment, and may be applied to any embodiment disclosed.

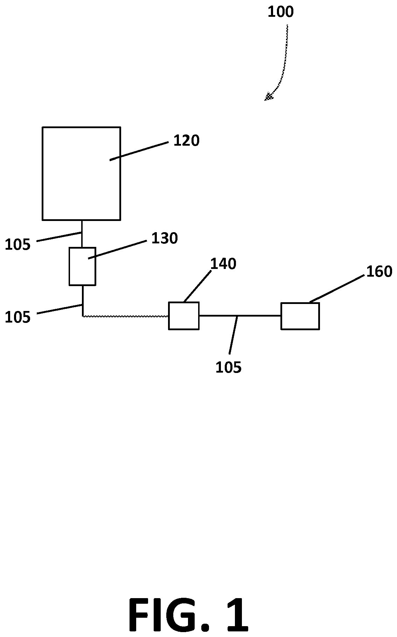

Turning to the drawings wherein like elements are described by similar reference numbers, FIG. 1 illustrates a sampling system 100 for collecting fluid samples from an enclosed container comprising at least one aseptic source 120, such as a bioreactor or similar container or system that supports biologically and/or chemically active environments. The sampling system also comprises at least one aseptic sampling system 130, a sample-directing manifold 140, and at least one analytical device 160. The at least one aseptic source 120, the at least one aseptic sampling system 130, the sample directing manifold 140, and at least one analytical device 160 are in fluid communication along a fluid flow path 105.

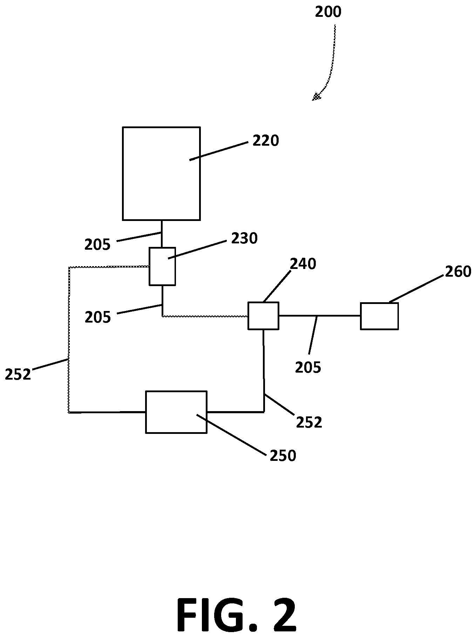

FIG. 2 illustrates another embodiment of the sampling system. FIG. 2 is similar to FIG. 1, but with the addition of a control module 250, that is in communication with the at least one aseptic sampling system 230 and a sample-directing manifold 240, through line 252.

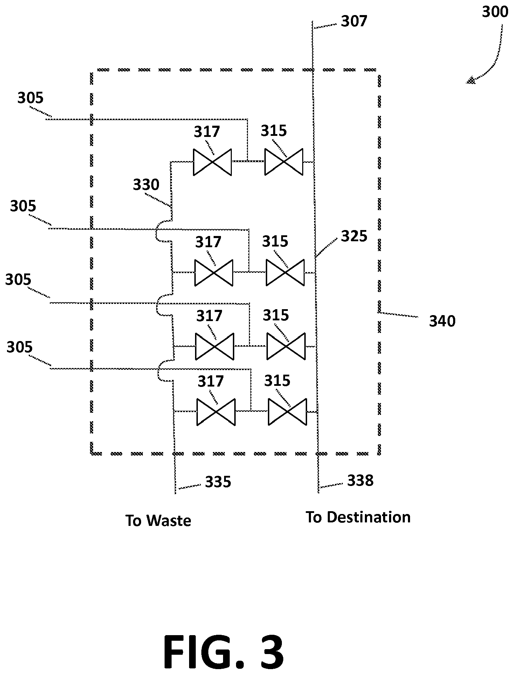

FIG. 3 illustrates a schematic of the sample-directing manifold 300. The sample-directing manifold 340 of FIG. 3 includes four sample inlets 305, each sample inlet 305 having a sample outlet valve 315 and a waste outlet valve 317. The sample-directing manifold also includes a flushing fluid inlet 307 that is upstream of the first sample outlet valve 315, a sample outlet path 325, and a sample outlet 338 that is downstream of the fourth sample outlet valve 315. The sample inlet 305 is located between the corresponding sample outlet valve 315 and waste outlet valve 317. The sample-directing manifold also includes a waste outlet path 330, and a waste outlet 335 that is downstream of the last waste outlet valve 317.

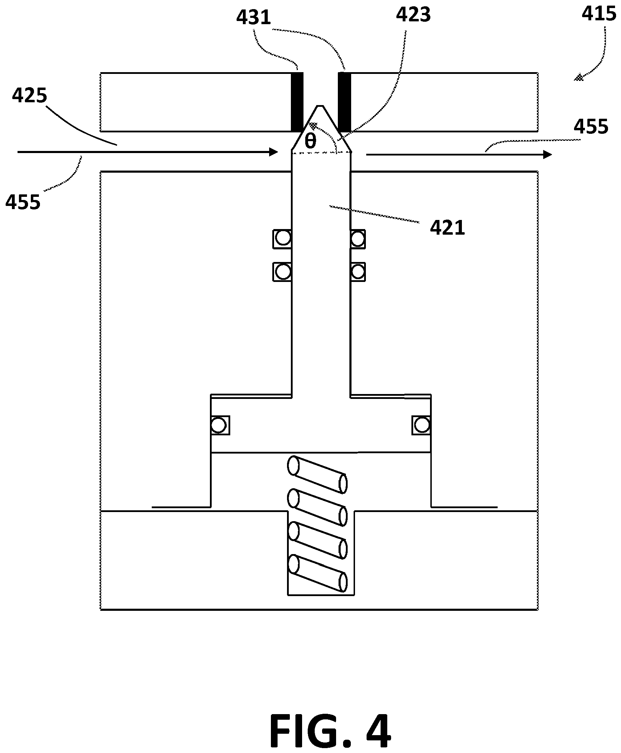

FIG. 4 illustrates an enlarged partial cross-sectional view of a sample outlet valve 415. When the sample outlet valve is in a closed position sanitant 455 can flow around the end of valve stem 421. Sealing member 423 seals the valve into seat 431. Thus, for example, as shown by arrows 455, sanitant can pass around a portion of the valve stem 421, thereby improving sanitization or sterilization of the sample outlet path 425.

The waste outlet valves 317 are of a similar design as shown in FIG. 4, and can be sanitized using a similar procedure.

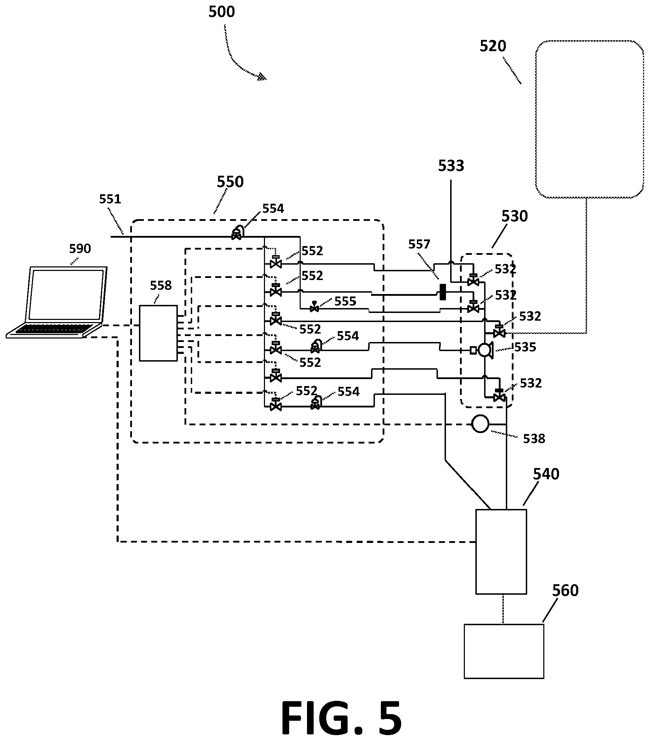

FIG. 5 shows the sampling system 500. FIG. 5 comprises an aseptic source 520, a control module 550, an aseptic sampling valve 530, a sample-directing manifold 540, and an analytical device 560. The control module comprises a gas source 551, a plurality of control valves 552, at least two pressure regulators 554, valve 555, and gas filter 557. The control module also includes a programmable logic controller (PLC) 558 that communicates with the components in the control module 550. A computer interface 590 is also shown for controlling the sampling system 500. The aseptic sampling valve 530 comprises valves 532 and a sample pump 535. The sample pump 535 can be a variable volume sample pump or a variable volume reservoir.

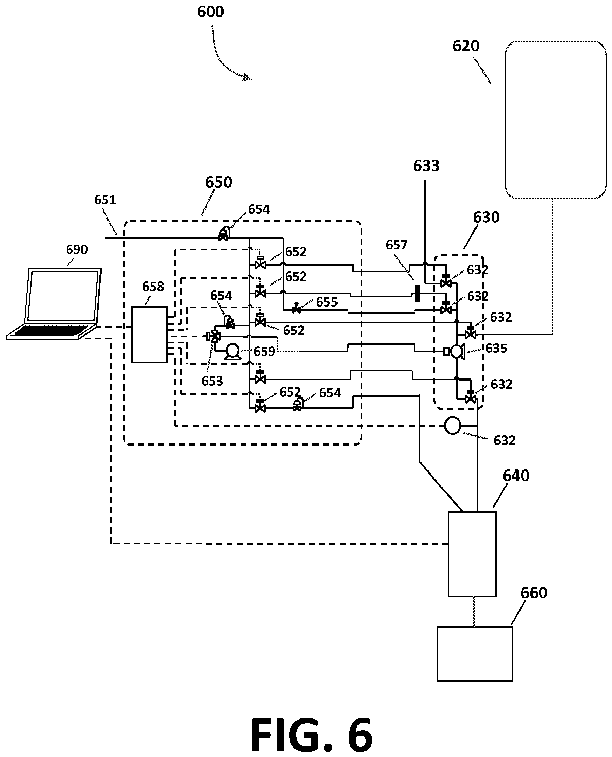

FIG. 6 shows an aseptic source 620, a control module 650, and aseptic sampling valve 630, a sample-directing manifold 640, and an analytical device 660. The control module 650 comprises a gas source 651, a plurality of control valves 652, at least three pressure regulators 654, valve 655, gas filter 657, and vacuum pump 659. The control module also includes a programmable logic controller (PLC) 658 that communicates with the components in the control module. A computer interface 690 is also shown for controlling the sampling system 600. The aseptic sampling valve 630 comprises valves 632 and a sample pump 635. The sample pump 635 is a variable volume sample pump or a variable volume reservoir.

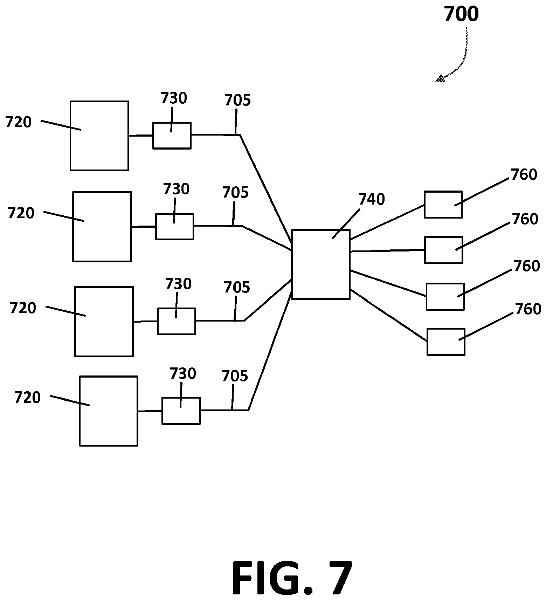

In Fig. 7, the sampling system 700 comprises four aseptic sources 720, four aseptic sampling systems 730 in fluid communication with the corresponding aseptic sources 720. Sampling system 700 also comprises sample-directing manifold 740, in fluid communication with corresponding sample inlets 705. Sampling system 700 also comprises four analytical devices 760, in fluid communication with the sample-directing manifold 740. The sampling system 700 can be used to direct sample from each of the aseptic sources 720 to the sample-directing manifold 740 using the aseptic sampling systems 730, and direct the sample to any of the analytical devices 760, using the control module (not shown in FIG. 7).

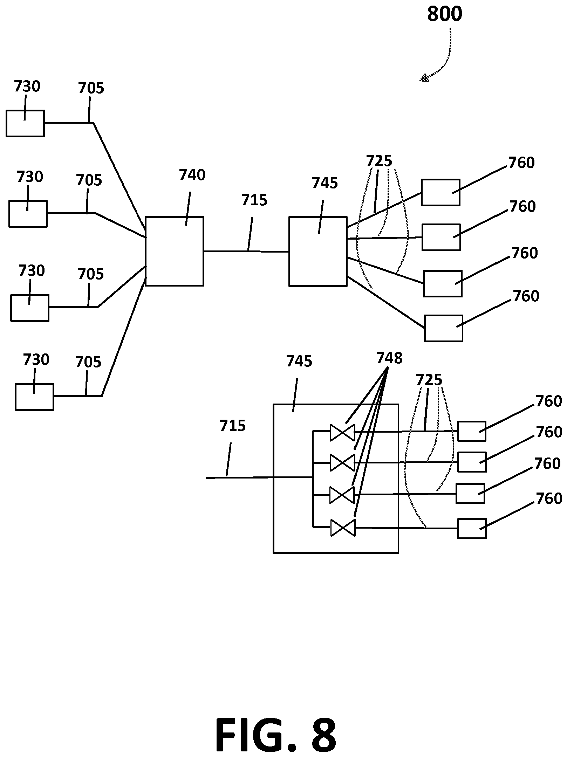

FIG. 8 shows four aseptic sampling systems 730 in fluid communication sample-directing manifold 740 through corresponding sample inlets 705. Sample-directing manifold 740 is in communication with analytical-directing manifold 745. Analytical-directing manifold contains four sample-directing valves 748 in fluid communication through lines 725 to four analytical devices 760. In one embodiment, the sampling system 800 may be configured independently wherein the sample-directing valves 748 may be in fluid communication with only one analytical device 760. In another embodiment, the sampling system 800 may be configured independently wherein sample-directing valves 748 may be in fluid communication with two or more analytical devices 760.

The sanitant is any fluid that can sanitize, disinfect, or sterilize the sampling module. The sanitant can be a liquid, a gas, or a combination thereof. Sanitants include steam, ethylene oxide, glutaraldehyde, formaldehyde, formalin, chlorine gas, hypochlorite, bromine, hypobromite, iodine, hypoiodite, bromine chloride, chlorine dioxide, ozone, hydrogen peroxide, monochloramine, dichloramine, trichloramine, quaternary ammonium salts, ethanol, 70% ethanol/water, isopropanol, 70% isopropanol/water, peroxyacetic acid, and peracetic acid. This list of possible sanitants should not be construed to indicate that all alternatives are equivalent to one another. The sanitant may be steam, ethylene oxide, glutaraldehyde, ethanol or a mixture of ethanol and water such as 70% ethanol/water.

The gas may be air, nitrogen, or any gas appropriate to purge the sanitant and sample from the sampling module. The gas is filtered through an appropriate filter to remove contaminants that may affect the aseptic nature of the samples. The gas may be nitrogen or air.

Referring to FIG. 4, the sample outlet valve 415 comprises a valve stem 421, a sealing member 423, and a seat 431. FIG. 4 illustrates that valve stem 421 tapers at the end to form sealing member 423. To provide improved sealing characteristics, the tip of the valve stem (sealing member 423) can extend at an angle (θ in FIG. 4) of greater than 50 degrees from the body of the valve stem 421 or the taper is an angle of greater than 60 degrees, the taper is an angle of greater than 70 degrees, or the taper is an angle of about 80 degrees.

The sealing member 423 can be formed of a material that has a lower yield strength than the material of the seat 431, into which sealing member 423 extends. In some embodiments, sealing member 423 can be made of metals, thermoplastic polymers, such as polyether-ether ketone (PEEK), polyether imide (PEI), polyphenylsulfone (PPSU), polysulfone (PSU), or combinations of these. The sealing member 423 is made from creep resistant metals, such as stainless steel, titanium, nickel, brass, and anodized aluminum; or high temperature thermoplastic polymers such as PEEK, or PPSU (also known as Radel®). The seat 431 can be made from a more flexible material, such as silicone rubber, polytetrafluoroethylene (PTFE, also known as Teflon®), perfluroalkoxy (PFA), ethylene tetrafluoroethylene (ETFE), high density polyethylene (HDPE), high density polypropylene (HDPP), perfluroalkoxy (PFA, also known as Viton®), and combinations thereof. In one embodiment, the valve stem 421 and the sealing member 423 are selected from metals, PEEK, PPSU, PEI, and mixtures thereof. Suitable metals include stainless steel, titanium, nickel, brass, and anodized aluminum. In one embodiment the seat is selected from PFA, PTFE, HDPE, HDPP, PFA and mixtures thereof. This list is not intended to indicate that each alternative is necessarily equivalent to the others.

The sealing member 423 is selected from PEEK, PPSU, and PEI, and the seat 431 material is selected from PTFE, Teflon®, ETFE, HDPE, HDPP, PFA, due to their relatively chemically inert behavior.

The sealing member and seat are arranged to form a variable sealing area when the sealing member and seat are in contact, causing the seat 431 material to deform until the stress on the materials at the seal is within the elastic modulus of the seat material, allowing a good seal even with relatively wide tolerances on the angles of the seat 431 and sealing member 423.

The sealing member 423 is formed of a higher yield strength material, such as a polymeric material, such as PTFE, PFA, ETFE, HDPE, HDPP, etc., while the seat 431 is formed of a more lower yield strength material, such as thermoplastic polymers (PEEK, PEI, PPSU, PSU, etc.), metals, or combinations or these materials. In this embodiment, sealing member 423 can extrude into the seat 431 to form a tight seal. In addition, in one embodiment, sealing member 423 and seat 431 can be cone shaped and may be inversely shaped such that the cone seat 431 comprises a hollow cone into which the cone shaped member 423 may fit. The sealing member 423 can have a steeper cone shape than the hollow cone seat 431, thereby allowing sealing member 423 to extrude into the seat 431 to form a positive seal.

When one or both of the sealing member 423 and seat 431 are formed of polymers, the heat up and cool down times associated with those parts can be faster than the times associated with other materials, such as steel or other metals.

The sealing member and valve stem can be formed of the same polymeric material, which can further improve operation by reducing complexities of manufacturing and permitting the sealing member and valve stem component to be more compact.

The sample-directing manifold comprises 2 module inlets and associated sample outlet valves and waste outlet valves. The sample-directing manifold may comprise 3 module inlets and associated sample outlet valves and waste outlet valves. The sample-directing manifold may comprise 4 module inlets and associated sample outlet valves and waste outlet valves. At least two sample-directing manifolds may be combined to one or more analytical devices or other destinations.

The analytical device may be a pH meter, a dissolved oxygen probe, an osmometer, high-performance liquid chromatograph (HPLC), a conductivity meter, a gas chromatograph, a mass spectrometer, ion chromatography, dielectric spectroscopy, microscopy, quantitative visualization tools, focused beam reflectance measurements (FBRM), particle vision and measurement (PVM) devices, a turbidity meter, reduction-oxidation probes, a flow cytometer, Raman/NIR spectroscopy, automated hemocytometer, electro-rotation, electrophoresis, dielectrophoresis, fluorescent activated cell sorting (FACS), or other analytical instruments designed to measure the properties of the aseptic fluid.

The sample-directing manifold comprises, consists of, or essentially consists of, a plurality of sample inlets, wherein each sample inlet is in fluid communication with a respective sample outlet valve and a respective waste outlet valve, a sample outlet path in fluid communication with each said respective sample outlet valve, and a waste outlet path in fluid communication with each said respective waste outlet valve. The respective waste outlet valve and each of the respective sample outlet valves may be configured independently so that only one of said plurality of sample inlets may be in fluid communication with said sample outlet path. In other embodiments, the sample-directing manifold as disclosed immediately above further comprise a flushing fluid inlet in fluid communication with said sample outlet path. The sample-directing is made from a body having internal channels defining said plurality of sample inlets, said sample outlet path, and said waste outlet path. The body having channels for each of said respective sample outlet valves and each of said respective waste outlet valves.

The sampling system for collecting a fluid sample from an enclosed container comprises the sample-directing manifold disclosed above. The sampling system may further comprises an aseptic sampling valve. The aseptic sampling valve may comprise a variable volume reservoir.

The sampling systems described above also may further comprise an control module in communication with said sample-directing manifold and said aseptic sampling valve.

The sample manifold comprises a plurality of sample inlets, each sample inlet having a sample outlet valve and a waste outlet valve, a sample outlet path, a waste outlet path, a gas inlet valve, a fluid inlet valve, and an outlet path isolation valve, wherein said sample manifold can be configured so that each sample inlet can be connected to said sample outlet path through said sample outlet valve while said outlet path isolation valve is closed, while the other sample inlets are connected to said waste outlet path through said waste outlet valves, and wherein said fluid inlet can be configured to remove said sample from said sample outlet path when said outlet path isolation valve is open so as to direct said fluid to waste, and said gas inlet can be used to remove said fluid from said outlet path when said isolation valve is open, so as to prepare said outlet path for receiving another sample.

The sampling system for collecting a fluid sample from an enclosed container comprises at least two modules, a control module, and a sampling module, wherein said control module comprises a source of compressed gas, one or more valves for directing compressed gas to the sampling module, and an optional vacuum pump; and the sampling module comprises a sanitizing fluid inlet valve, a gas inlet valve, a sample collection valve, an outlet valve, a variable volume reservoir, and a fluid flow path interconnecting the sanitizing fluid inlet valve, the gas inlet valve, the sample collection valve and the variable volume reservoir.

Also disclosed is a method of collecting a fluid sample from an aseptic container, comprising opening a sanitizing fluid inlet valve and directing sanitizing fluid through a sample outlet path, discharging said sanitizing fluid through an outlet path isolation valve, closing the sanitizing fluid inlet valve, opening a gas inlet valve to remove sanitant from the sample outlet path, and opening a sample inlet and a sample outlet valve to direct the sample to an analytical device.

The scope of the invention is defined by the following claims.

## Claims

1. A sampling system for collecting a fluid sample from an enclosed container comprising a sample-directing manifold and an aseptic sampling valve, wherein the sample-directing manifold comprises:
(a) a plurality of sample inlets, wherein each sample inlet is in fluid communication with a respective sample outlet valve and a respective waste outlet valve;
(b) a sample outlet path in fluid communication with each said respective sample outlet valve; and
(c) a waste outlet path in fluid communication with each said respective waste outlet valve
wherein each said respective waste outlet valve and each said respective sample outlet valves may be configured independently so that only one of said plurality of sample inlets may be in fluid communication with said sample outlet path.

2. The sampling system of claim 1, wherein the sample-directing manifold further comprises a flushing fluid inlet in fluid communication with said sample outlet path.

3. The sampling system of any of the previous claims wherein the sample-directing manifold is made from a body, said body having internal channels defining said plurality of sample inlets, said sample outlet path, and said waste outlet path.

4. The sampling system of claim 3, wherein said body comprises channels for each of said respective sample outlet valves and each of said respective waste outlet valves.

5. The sampling system of claim 1, wherein said aseptic sampling valve comprises a variable volume reservoir.

6. The sampling system of any of the preceding claims, further comprising a control module in communication with said sample-directing manifold and said aseptic sampling valve.

7. The sampling system of any of the preceding claims, wherein said sample manifold further comprises:
(a) a gas inlet valve,
(b) a fluid inlet valve, and
(c) an outlet path isolation valve;
wherein said sample manifold can be configured so that each sample inlet can be connected to said sample outlet path through said sample outlet valve while said outlet path isolation valve is closed, while the other sample inlets are connected to said waste outlet path through said waste outlet valves, and
wherein said fluid inlet can be configured to remove said sample from said sample outlet path when said outlet path isolation valve is open so as to direct said fluid to waste, and said gas inlet can be used to remove said fluid from said outlet path when said isolation valve is open, so as to prepare said outlet path for receiving another sample.

8. A sampling system according to any of the preceding claims for collecting a fluid sample from an enclosed container comprising at least two modules, a control module, and a sampling module;
(A) wherein said control module comprises:
(1) a source of compressed gas,
(2) one or more valves for directing compressed gas to the sampling module, and
(3) an optional vacuum pump; and
(B) said sampling module comprises:
(4) a sanitizing fluid inlet valve;
(5) a gas inlet valve;
(6) a sample collection valve;
(7) an outlet valve;
(8) a variable volume reservoir; and
(9) a fluid flow path interconnecting (4) - (8).

9. A method of collecting a fluid sample from an aseptic container, comprising:
providing a sampling system according to any of the preceding claims;
opening a sanitizing fluid inlet valve and directing sanitizing fluid through a sample outlet path, discharging said sanitizing fluid through an outlet path isolation valve; and
closing the sanitizing fluid inlet valve, opening a gas inlet valve to remove sanitant from the sample outlet path; opening a sample inlet and a sample outlet valve to direct the sample to an analytical device.

## Patentansprüche

1. Probeentnahmesystem zum Sammeln einer flüssigen Probe aus einem geschlossenen Behälter, umfassend einen Probe lenkenden Verteiler und ein aseptisches Probeentnahmeventil, wobei der Probe lenkende Verteiler Folgendes umfasst:
(a) mehrere Probeneinlässe, wobei jeder Probeneinlass in flüssiger Kommunikation mit einem jeweiligen Probenauslassventil und einem jeweiligen Abfallauslassventil steht;
(b) eine Probenauslassbahn in flüssiger Kommunikation mit jedem jeweiligen Probenauslassventil und
(c) eine Abfallauslassbahn in flüssiger Kommunikation mit jedem jeweiligen Abfallauslassventil,
wobei jedes jeweilige Abfallauslassventil und jede jeweiligen Probenauslassventile unabhängig konfiguriert sein können, sodass nur einer der mehreren Probeneinlässe in flüssiger Kommunikation mit der Probenauslassbahn stehen kann.

2. Probeentnahmesystem nach Anspruch 1, wobei der Probe lenkende Verteiler ferner einen Spülflüssigkeitseinlass in flüssiger Kommunikation mit der Probenauslassbahn umfasst.

3. Probeentnahmesystem nach einem der vorstehenden Ansprüche, wobei der Probe lenkende Verteiler aus einem Körper besteht, und der Körper innere Kanäle aufweist, die die mehreren Probeneinlässe, die Probenauslassbahn und die Abfallauslassbahn definieren.

4. Probeentnahmesystem nach Anspruch 3, wobei der Körper Kanäle für jedes der jeweiligen Probenauslassventile und jedes der jeweiligen Abfallauslassventile umfasst.

5. Probeentnahmesystem nach Anspruch 1, wobei das aseptische Probeentnahmeventil ein Speichergefäß mit variablem Volumen umfasst.

6. Probeentnahmesystem nach einem der vorstehenden Ansprüche, ferner umfassend ein Steuermodul in Kommunikation mit dem Probe lenkenden Verteiler und dem aseptischen Probeentnahmeventil.

7. Probeentnahmesystem nach einem der vorstehenden Ansprüche, wobei der Probenverteiler ferner Folgendes umfasst:
(a) ein Gaseinlassventil,
(b) ein Flüssigkeitseinlassventil und
(c) ein Auslassbahnisolierventil;
wobei der Probenverteiler so konfiguriert sein kann, dass jeder Probeneinlass an die Probenauslassbahn durch das Probenauslassventil angeschlossen werden kann, während das Auslassbahnisolierventil geschlossen ist, während die anderen Probenauslässe durch die Abfallauslassventile an die Abfallauslassbahn angeschlossen sind, und
wobei der Flüssigkeitseinlass konfiguriert sein kann, um die Probe aus der Probenauslassbahn zu entfernen, wenn das Auslassbahnisolierventil offen ist, um die Flüssigkit zum Abfall zu lenken und der Gaseinlass verwendet werden kann, um die Flüssigkeit aus der Auslassbahn zu entfernen, wenn das Isolierventil offen ist, um die Auslassbahn für den Empfang einer weiteren Probe vorzubereiten.

8. Probeentnahmesystem nach einem der vorstehenden Ansprüche zum Sammeln einer flüssigen Probe aus einem geschlossenen Behälter, umfassend mindestens zwei Module, ein Steuermodul und ein Probeentnahmemodul;
(A) wobei das Steuermodul Folgendes umfasst:
(1) eine Druckgasquelle,
(2) ein oder mehrere Ventile, um Druckgas zum Probeentnahmemodul zu lenken,
(3) eine wahlweise Vakuumpumpe; und
(B) wobei das Probeentnahmemodul Folgendes umfasst:
(4) ein Einlassventil für desinfizierende Flüssigkeit;
(5) ein Gaseinlassventil;
(6) ein Probensammelventil;
(7) ein Auslassventil;
(8) ein Speichergefäß mit variablem Volumen und
(9) eine (4) - (8) verbindende Flüssigkeitsstrombahn.

9. Verfahren zum Sammeln einer flüssigen Probe aus einem aseptischen Behälter, umfassend:
Bereitstellen eines Probeentnahmesystems nach einem der vorstehenden Ansprüche;
Öffnen eines sterilisierenden Flüssigkeitseinlassventils und Lenken der desinfizierenden Flüssigkeit durch eine Probenauslassbahn, Entladen der desinfizierenden Flüssigkeit durch ein Auslassbahnisolierventil und
Schließen des desinfizierenden Flüssigkeitseinlassventils, Öffnen eines Gaseinlassventils, um das Desinfektionsmittel aus der Probenauslassbahn zu entfernen; Öffnen eines Probeneinlass- und eines Probenauslassventils, um die Probe zu einer analytischen Vorrichtung zu lenken.

## Revendications

1. Système d'échantillonnage pour prélever un échantillon de fluide à partir d'un récipient fermé comprenant un collecteur de prélèvement d'échantillon et une vanne d'échantillonnage aseptique, dans lequel le collecteur de prélèvement d'échantillon comprend:
(a) une pluralité d'entrées d'échantillon, chaque entrée d'échantillon étant en communication fluidique avec une vanne de sortie d'échantillon correspondante et une vanne de sortie de déchets correspondante;
(b) un trajet de sortie d'échantillon en communication fluidique avec chacune desdites vannes de sortie d'échantillon correspondantes; et
(c) un trajet de sortie de déchets en communication fluidique avec chacune desdites vannes de sortie de déchets correspondantes
dans lequel chacune desdites vannes de sortie de déchets correspondantes et chacune desdites vannes de sortie d'échantillon correspondantes peuvent être configurées indépendamment de sorte qu'une seule entrée de ladite pluralité d'entrées d'échantillon peut être en communication fluidique avec ledit trajet de sortie d'échantillon.

2. Système d'échantillonnage selon la revendication 1, dans lequel le collecteur de prélèvement d'échantillon comprend en outre une entrée de fluide de rinçage en communication fluidique avec ledit trajet de sortie d'échantillon.

3. Système d'échantillonnage selon l'une quelconque des revendications précédentes, dans lequel le collecteur de prélèvement d'échantillon est réalisé à partir d'un corps, ledit corps ayant des canaux internes définissant ladite pluralité d'entrées d'échantillon, ledit trajet de sortie d'échantillon, et ledit trajet de sortie de déchets.

4. Système d'échantillonnage selon la revendication 3, dans lequel ledit corps comprend des canaux pour chacune desdites vannes de sortie d'échantillon correspondantes et chacune desdites vannes de sortie de déchets correspondantes.

5. Système d'échantillonnage selon la revendication 1, dans lequel ladite vanne d'échantillonnage aseptique comprend un réservoir à volume variable.

6. Système d'échantillonnage selon l'une quelconque des revendications précédentes, comprenant en outre un module de commande en communication avec ledit collecteur de prélèvement d'échantillon et ladite vanne d'échantillonnage aseptique.

7. Système d'échantillonnage selon l'une quelconque des revendications précédentes, dans lequel ledit collecteur d'échantillons comprend en outre:
a) une vanne d'entrée de gaz,
b) une vanne d'entrée de fluide, et
c) une vanne d'isolement du trajet de sortie;
dans lequel ledit collecteur d'échantillons peut être configuré de telle sorte que chaque entrée d'échantillon puisse être connectée audit trajet de sortie d'échantillon à travers ladite vanne de sortie d'échantillon pendant que ladite vanne d'isolement du trajet de sortie est fermée, tandis que les autres entrées d'échantillon sont connectées audit trajet de sortie de déchets à travers lesdites vannes de sortie de déchets, et
dans lequel ladite entrée de fluide peut être configurée pour retirer ledit échantillon dudit trajet de sortie d'échantillon lorsque ladite vanne d'isolement du trajet de sortie est ouverte de manière à diriger ledit fluide vers les déchets, et ladite entrée de gaz peut être utilisée pour retirer ledit fluide dudit trajet de sortie lorsque ladite vanne d'isolement est ouverte, de manière à préparer ledit trajet de sortie pour recevoir un autre échantillon.

8. Système d'échantillonnage selon l'une quelconque des revendications précédentes pour prélever un échantillon de fluide à partir d'un récipient fermé comprenant au moins deux modules, un module de commande et un module d'échantillonnage;
(A) dans lequel ledit module de commande comprend:
(1) une source de gaz comprimé,
(2) une ou plusieurs vannes pour diriger le gaz comprimé vers le module d'échantillonnage, et
(3) une pompe à vide optionnelle; et
(B) ledit module d'échantillonnage comprend:
(4) une vanne d'entrée de fluide désinfectant;
(5) une vanne d'entrée de gaz;
(6) une vanne de prélèvement d'échantillon;
(7) une vanne de sortie;
(8) un réservoir à volume variable; et
(9) un trajet d'écoulement de fluide reliant (4) et (8).

9. Procédé de prélèvement d'un échantillon de fluide à partir d'un récipient aseptique, consistant à:
fournir un système d'échantillonnage selon l'une quelconque des revendications précédentes;
ouvrir une vanne d'entrée de fluide désinfectant et diriger le fluide désinfectant à travers un trajet de sortie d'échantillon, et décharger ledit fluide désinfectant à travers une vanne d'isolement du trajet de sortie; et
fermer la vanne d'entrée du fluide désinfectant, et ouvrir une vanne d'entrée de gaz pour retirer le produit désinfectant du trajet de sortie de l'échantillon;
ouvrir une vanne d'entrée d'échantillon et une vanne de sortie d'échantillon pour diriger l'échantillon vers un dispositif d'analyse.
